(19) Europäisches Patentamt
European Patent Office
Office européen des brevets

(11) **EP 3 231 481 A1**

(12) **EUROPEAN PATENT APPLICATION**

(43) Date of publication:
**18.10.2017 Bulletin 2017/42**

(51) Int Cl.:
***A61N 5/10*** *(2006.01)*   ***A61B 6/00*** *(2006.01)*

(21) Application number: **17157599.6**

(22) Date of filing: **23.02.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **15.04.2016   JP 2016082278
29.12.2016   US 201615394332**

(71) Applicant: **Kabushiki Kaisha Toshiba
Minato-ku,
Tokyo (JP)**

(72) Inventors:
• **Taguchi, Yasunori
Tokyo (JP)**
• **Hirai, Ryusuke
Tokyo (JP)**
• **Sakata, Yukinobu
Tokyo (JP)**

(74) Representative: **Hoffmann Eitle
Patent- und Rechtsanwälte PartmbB
Arabellastraße 30
81925 München (DE)**

(54) **PROCESSING DEVICE FOR A RADIATION THERAPY SYSTEM**

(57)    A processing device (20) for a radiation device (10) is configured to carry out the steps of retrieving, from a data storage (50), volume data of a subject that was generated by imaging an internal structure of the subject, determining a position of an object in the subject based on the retrieved volume data of the subject, obtaining geometry information including a position of a radiation source (112a, 112b) and a position of a radiation detector (116a, 116b), and obtaining a direction of the radiation detector, and determining a condition for imaging with the radiation source, so that the object can be captured through the imaging, based on the volume data, the position of the object, the position of the radiation source, the position of the radiation detector, and the direction of the radiation detector.

*FIG. 1*

## Description

FIELD

Arrangement described herein relate generally to a processing device for a radiation therapy system.

BACKGROUND

Generally, as radiation therapy methods for a part of a patient's body that moves due to breath, heartbeat, and moving of intestine and the like, a gated irradiation method and a pursuing irradiation method are known.

According to these irradiation methods, fluoroscopic images that contain an affected part of the patient's body or a marker located at or around the affected part are regularly generated by regularly irradiating the affected part of the patient with radiation for X-ray fluoroscopy. A position of the affected part or the marker is tracked using the fluoroscopic images, and radiation for treatment is applied to the affected part. When the fluoroscopic images are captured in two or more directions, a three-dimensional position of the affected part or the marker can be determined. However, the amount of exposure of the patient to the radiation for X-ray fluoroscopy cannot be ignored.

In the related art, an irradiation range of the X-ray for fluoroscopy is calculated based on a position of a marker detected from a fluoroscopic image that was captured previously. For that reason, the irradiation range of the X-ray for fluoroscopy can be limited to a narrow range corresponding to the position of the marker. However, in order to determine the narrow irradiation range, an irradiation range of the fluoroscopic image that was captured previously will need to be wider to ensure the marker position in the image.

BRIEF DESCRIPTION OF THE DRAWINGS

FIG. 1 illustrates a radiation therapy system according to a first arrangement.
FIG. 2 is a flow chart illustrating an operation of the radiation therapy system according to the first arrangement.
FIG. 3 illustrates an example of a DRR (digitally reconstructed radiograph) corresponding to a fluoroscopic image captured by an X-ray detector of the radiation therapy system.
FIG. 4 illustrates an example of a DRR corresponding to a fluoroscopic image captured by another X-ray detector of the radiation therapy system.
FIG. 5 illustrates an example of an irradiation range in the DRR depicted in FIG. 3.
FIG. 6 illustrates an example of an irradiation range in the DRR depicted in FIG. 4.

FIG. 7 schematically illustrates a moving image of the DRR depicted in FIG. 3 to explain a method for determining a condition for fluoroscopic imaging when three-dimensional volume data is moving image data.
FIG. 8 schematically illustrates a moving image of the DRR depicted in FIG. 4 to explain a method for determining the condition for fluoroscopic imaging when the three-dimensional volume data is moving image data.
FIG. 9 illustrates an irradiation range when one object on the DRR depicted in FIG. 3 is a tracking target.
FIG. 10 illustrates an irradiation range when one object on the DRR depicted in FIG. 4 is a tracking target.
FIG. 11 exemplifies a graphical user interface displayed on a display of the radiation therapy system.
FIGS. 12A and 12B illustrate an example of a contour of an object.

DETAILED DESCRIPTION

An arrangement is directed to providing a processing device for a radiation therapy system which reduces the amount of unnecessary radiation to which a patient is exposed.

In general, according to one arrangement, a processing device (20) for a radiation device (30), that is configured to carry out the steps of retrieving, from a data storage (50), volume data of a subject that was generated by imaging an internal structure of the subject, determining a position of an object in the subject based on the retrieved volume data of the subject, obtaining a position a position of a radiation source (112a, 112b), a position of a radiation detector (116a, 116b), and a direction of the radiation detector, and determining a condition for imaging with the radiation source, so that the object can be captured through the imaging, based on the volume data, the position of the object, the position of the radiation source, the position of the radiation detector, and the direction of the radiation detector.

Hereinafter, embodiments will be described. Also, the same configurations or processes for performing the same operation are described with the same number, and description thereof will not be repeated.

(First Embodiment)

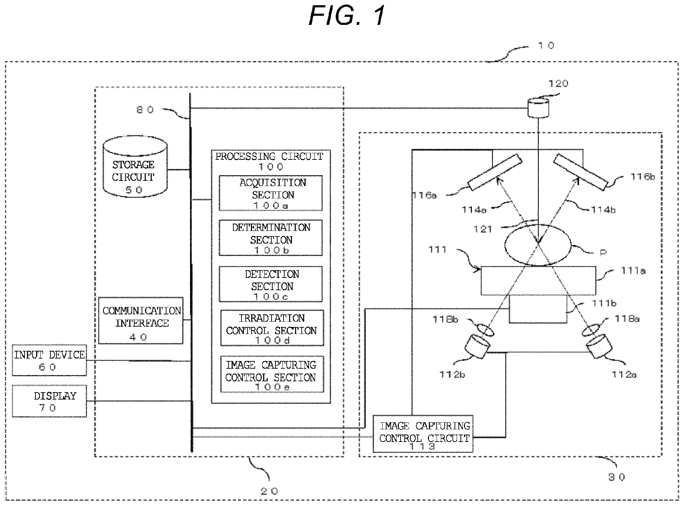

FIG. 1 illustrates an example of a radiation therapy system 10 according to a first embodiment. The radiation therapy system 10 is a system for treating a subject P with radiation 121 for treatment. In the embodiments hereinafter, X-ray is assumed to be used as radiation for fluoroscopy, and a heavy particle beam is assumed to be used as the radiation 121 for treatment. However, in the present disclosure, the radiation for fluoroscopy and the radiation 121 for treatment may be any of an X-ray, a y-ray, an electron beam, a proton beam, a neutron beam, a heavy particle beam, and the like.

Image capturing for detecting motion is often referred to as a perspective imaging, but the image capturing for detecting motion is simply referred to as "image capturing" herein, without any further distinction.

The radiation therapy system 10 includes an information processing apparatus 20, an input device 60, a display 70, a fluoroscopic imaging device 30, and a radiation port 120.

The information processing apparatus 20 performs various kinds of image processing using fluoroscopic images captured by the fluoroscopic imaging device 30, and controls the amount of the radiation 121 for treatment, which is emitted from the radiation port 120. The information processing apparatus 20 is, either, a special-purpose computer or a general-purpose computer. The information processing apparatus 20 may be, for example, a PC (work station) connected to the fluoroscopic imaging device 30 and the radiation port 120 through a network, or may be an information processing apparatus included in a server for storing and managing medical images. The medical images include images captured by various image diagnosis apparatuses such as a computed tomography (CT) apparatus, a magnetic resonance imaging apparatus (MRI), a X-ray image, and a fluoroscopic image.

Specifically, the information processing apparatus 20 includes a processing circuit 100, a storage circuit 50, a communication interface 40, and a bus 80 that connects the components of the image processing apparatus.

In one embodiment, the processing circuit 100 is a processor, such as a central processing unit (CPU), a graphical processing unit (GPU). In other embodiments, the processing circuit 100 may be any of the following logic devices, e.g., an application specific integrated circuit (ASIC), a programmable logic device (for example, simple programmable logic device (SPLD)), a complex programmable logic device (CPLD), and a field programmable gate array (FPGA). The processing circuit 100 includes, as functional sections, an acquisition section 100a, a determination section 100b, a detection section 100c, an irradiation control section 100d, and an image capturing control section 100e. These functional sections will be described below in detail.

In the embodiment where the processing circuit 100 is a processor, the processor is executing programs stored in the storage circuit 50, and each of the functional sections represents the processor reading a corresponding program from the storage circuit 50 and executing the program. The steps of the program for each of the functional sections are described below in conjunction with the detailed descriptions of each functional section. Also, FIG. 1 illustrates that a single processing circuit 100 functions as the acquisition section 100a, the determination section 100b, the detection section 100c, the irradiation control section 100d, and the image capturing control section 100e. However, these functional sections may be configured in different processing circuits, each of which includes one of the functional sections.

Moreover, the acquisition section 100a and the determination section 100b of the processing circuit 100 are referred to herein as an acquiring unit and a determining unit, respectively.

In the embodiments where the processing circuit 100 is one of the logic devices, the logic device is configured to execute the functions of the acquisition section 100a, the determination section 100b, the detection section 100c, the irradiation control section 100d, and the image capturing control section 100e.

The storage circuit 50 stores data or the like according to processing of each functional section of the processing circuit 100 as needed. The storage circuit 50 according to the present embodiment stores programs and three-dimensional volume data of the subject P that was generated when a treatment plan was created. For example, the storage circuit 50 is a random access memory (RAM), a semiconductor memory element such as flash memory, a hard disk, an optical disk, and the like. In addition, processes performed by the storage circuit 50 of the information processing apparatus 20 may be performed by an external storage that is connected externally to the information processing apparatus 20. The storage circuit 50 may include a recording medium which permanently or temporally stores a program downloaded via a local area network (LAN), from the Internet or the like. In addition, the recording medium of the present embodiment is not limited to one and may be formed of a plurality of recording mediums.

The communication interface 40 is an interface which inputs and outputs information from and to an external device connected in a wired or wireless manner. The communication interface 40 may perform communication by being connected to a network.

The input device 60 receives various kinds of instructions and inputs from an operator. The input device 60 is, for example, an input device such as a pointing device, such as a mouse or a track ball, or a key board. The operator may be a doctor or an engineer, but not limited thereto.

The display 70 displays various kinds of information including images. The display 70 is, for example, a display device such as a liquid crystal display.

The input device 60 and the display 70 according to the present embodiment are connected to the information processing apparatus 20 in a wired or wireless manner. The input device 60 and the display 70 may be connected to the information processing apparatus 20 through a network.

The radiation port 120 emits the radiation 121 for treatment toward the subject P.

The fluoroscopic imaging device 30 includes X-ray sources 112a and 112b, collimators 118a and 118b, X-ray detectors 116a and 116b, a bed 111, and an image capturing control circuit 113.

The X-ray sources 112a and 112b respectively emit X-rays 114a and 114b.

The X-ray detectors 116a and 116b detect the X-rays 114a and 114b transmitted through the subject P, respectively. The X-ray detectors 116a and 116b are, for example, a flat panel detector or an image intensifier. The X-ray detectors 116a and 116b generate a fluoroscopic image from the detected X-rays 114a and 114b, respectively.

The collimators 118a and 118b are integrally disposed with the X-ray sources 112a and 112b, respectively. The collimators 118a and 118b are arranged closer to the X-ray detectors 116a and 116b with respect to the X-ray sources 112a and 112b, respectively. Each of the collimators are disposed between each of the X-ray sources 112a and 112b and the subject P. The collimators 118a and 118b control an irradiation range of the X-rays 114a and 114b, respectively.

The image capturing control circuit 113 controls positions of the X-ray sources 112a and 112b, the collimators 118a and 118b, and the X-ray detectors 116a and 116b with respect to the subject P. These positions are adjusted, so that an object is set within an angle of view, and captured clearly. In particular, the X-rays 114a and 114b are directed in different directions from each other.

The bed 111 includes a top board 111a where the subject P is laid down and a bed control circuit 111b. The bed control circuit 111b controls the top board 111a so that the subject P is located at a position determined in the treatment plan. When the subject P is a patient, this control is referred to as patient position determination. The top board 111a is moved in a longitudinal direction and a vertical direction in a state in which the subject P is laid down, under a control of the bed control circuit 111b. A moving direction of the top board 111a is not limited to the longitudinal and vertical directions. Translation in a three-dimensional space can be described with three parameters and rotation in the three-dimensional space can be described with three parameters, and thus any movement can be described with six parameters.

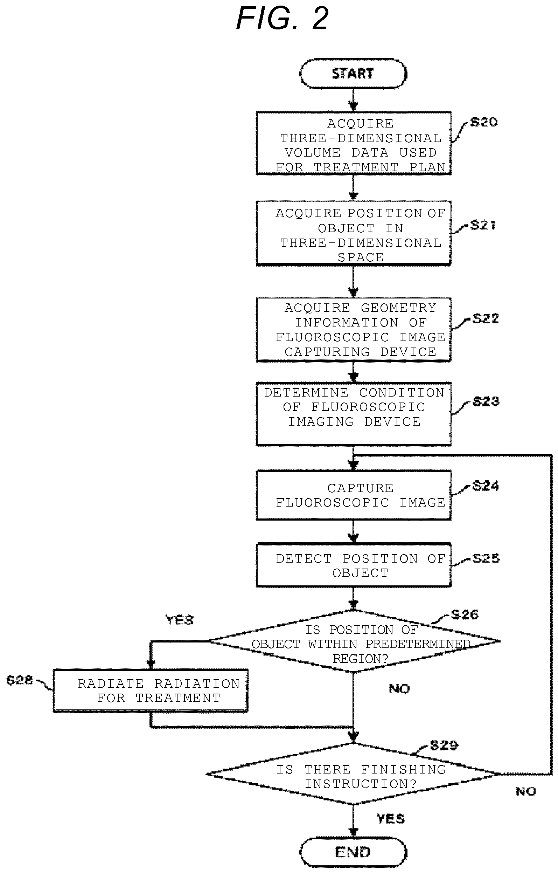

FIG. 2 is a flow chart illustrating a flow of a process carried out by the processing circuit 100 according to a first embodiment. With reference to FIG. 2, an operation by each functional section of the processing circuit 100 will be described.

The acquisition section 100a of the processing circuit 100 acquires the three-dimensional volume data of the subject P from the storage circuit 50, and the subject P is placed at a position determined in the treatment plan based on the three-dimensional volume data (Step S20). The three-dimensional volume data is, for example, the CT image data or the MRI image data of the subject P used to create the treatment plan. Alternatively, the acquisition section 100a of the processing circuit 100 may receive the three-dimensional volume data from outside the radiation therapy system 10 through the communication interface 40. The three-dimensional volume data may be any three-dimensional image information, and is not limited to the CT image data or the MRI image data.

The acquisition section 100a of the processing circuit 100 acquires the position of an object in a three-dimensional space corresponding to the three-dimensional volume data from the storage circuit 50 (Step S21). Here, the object is assumed to be a marker located at an affected part of the subject P or inside the body of the subject P. For example, the position of the object includes a center position and a position on the contour of the object. The acquisition section 100a of the processing circuit 100 acquires information of the position of the object that was input by the operator when the treatment plan was created. The contour of the object is typically input by the operator when the treatment plan was created whether the object is the affected part or the marker. The center may be obtained from the contour of the object. Instead, a center position of the object may be input by the operator in advance. Moreover, the acquisition section 100a of the processing circuit 100 may receive the position of the object from outside the radiation therapy system 10 through the communication interface 40. Alternatively, the acquisition section 100a of the processing circuit 100 may calculate the position of the object from the three-dimensional volume data. For example, when the three-dimensional volume data is the CT image data and the object as a tracking target in the fluoroscopic image is the marker, a position of a voxel value corresponding to a CT value unique to a material of the marker may be determined as a position of the marker. In addition, the position of the affected part or the marker may be specified by various image processing technologies.

The acquisition section 100a of the processing circuit 100 acquires data of the treatment plan and geometry information that indicates positions of the X-ray sources 112a and 112b and the X-ray detectors 116a and 116b of the fluoroscopic imaging device 30 in a treatment room (Step S22). The geometry information includes information to project position of the object from three-dimensional space onto two-dimensional space of image by a radiation source and a radiation detector. The geometry information may include at least one of a position of a radiation source, a position of a radiation detector, and a direction of the radiation detector. For example, the acquisition section 100a of the processing circuit 100 acquires the geometry information from the image capturing control circuit 113, and acquires the treatment plan data from the storage circuit 50. Alternatively, the acquisition section 100a of the processing circuit 100 may acquire the geometry information obtained in advance from the storage circuit 50 or from the outside of the radiation therapy system 10 through the communication interface 40.

The determination section 100b of the processing circuit 100 determines a condition for fluoroscopic imaging of the fluoroscopic imaging device 30 using the geometry information and the three-dimensional volume data (Step S23). A method of determining the condition for fluoroscopic imaging will be described below in detail.

The image capturing control section 100e of the processing circuit 100 controls the fluoroscopic imaging device 30 so that the fluoroscopic image is captured in accordance with the condition for fluoroscopic imaging determined by the determination section 100b (Step S24). The condition for fluoroscopic imaging includes at least one of, for example, an irradiation range, an irradiation intensity, and irradiation energy of the X-rays 114a and 114b, and an output current and an output voltage of the X-ray sources 112a and 112b. The irradiation ranges of the X-rays 114a and 114b are determined by positions of the collimators 118a and 118b, respectively. That is, the condition for fluoroscopic imaging corresponds to an irradiation condition of the X-rays 114a and 114b.

The detection section 100c of the processing circuit 100 detects the position of the object in the fluoroscopic image of the subject P captured by the fluoroscopic imaging device 30 (Step S25). To detect the object, various image processing technologies can be employed. For example, digitally reconstructed radiographs (DRRs) corresponding to the fluoroscopic images captured by the fluoroscopic imaging device 30 are generated from the three-dimensional volume data. Specifically, the DRRs are generated by virtually locating the three-dimensional volume data at a predetermined position determined relative to an isocenter of the treatment room as a reference position, according to the orientation of the subject P included in the treatment plan data, and using the geometry information. Here, the isocenter is a point in a three-dimensional space used as a reference to direct the radiation 121 for treatment. When the subject P is a human body, an orientation of the subject P determined in the treatment plan is, for example, a face-up orientation, a face-down orientation, and the like. Since the subject P is located at the position determined in the treatment plan in Step S20, an angle of view of the generated DRR becomes the same as the fluoroscopic image. By projecting the position of the object in the acquired three-dimensional volume data to the fluoroscopic image using the geometry information, the position of the object in the generated DRR and an image pattern of the object in the generated DRR may be calculated, and a position in the fluoroscopic image including similar patterns may be detected as a position of the object. Projection is illustrated by the following equation.

$$(x, y, 1)^T = P(X, Y, Z, 1)^T$$

Here, (x, y) indicates a coordinate value of the position of the object in the two-dimensional fluoroscopic image. P indicates a projection matrix determined from the geometry information. (X,Y,Z) indicates a coordinate value of the position of the object in the three-dimensional real world. "T" denotes transpose of a matrix or a vector.

When the position of the object in the detected fluoroscopic image is within a preset region (YES in Step S26), the irradiation control section 100d of the processing circuit 100 controls the radiation port 120 to emit the radiation 121 for treatment toward the subject P (Step S28). Irradiation condition of the radiation 121 for treatment is not limited to the above description.

When the detected position of the object in the fluoroscopic image is not within the preset region (YES in Step S26), the process proceed to Step S29.

When there is no instruction to finish the process (YES in S29), the process returns to Step S24. The instruction to finish the process may be input by the operator, or may be automatically generated when a condition relating to an amount of irradiation of the radiation 121 for treatment determined in the treatment plan is satisfied. When the instruction to finish is received (No in S29), the process is finished.

In the flow chart shown in FIG. 2, Step S20 to Step S29 may not be necessarily carried out in this order.

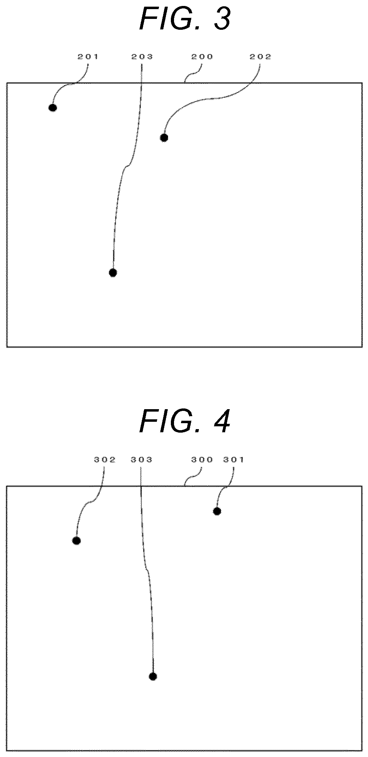

Next, referring to FIG. 3 to FIG. 10, a method of determining the condition for fluoroscopic imaging by the determination section 100b of the processing circuit 100 will be described in detail. First, as the condition for fluoroscopic imaging, a method of determining the irradiation range of the X-ray 114a and 114b will be described. Here, it is assumed that the object is a marker, but the condition for fluoroscopic imaging can be determined in the same manner when the object is an affected part.

The determination section 100b calculates the position of the object in the fluoroscopic image captured by the fluoroscopic imaging device 30 by overlaying the position of the object in the three-dimensional volume data to the fluoroscopic image using the geometry information. This calculation expressed by the equation $(x, y, 1)^T = P(X, Y, Z, 1)^T$ described above.

FIG. 3 is an example of a DRR 200 corresponding to a fluoroscopic image generated by the X-ray detector 116a.

FIG. 4 is an example of a DRR 300 corresponding to a fluoroscopic image generated by the X-ray detector 116b.

In these examples, three makers are located in the subject P. In the DRR 200, images 201, 202, and 203 of the markers are included. In the DRR 300, images 301, 302, and 303 of the markers are included. In these examples, the image 201 corresponds to the image 301 of the marker. The image 202 corresponds to the image 302 of the marker. The image 203 corresponds to the image 303 of the marker. When the DRR 200 and the DRR 300 are generated and displayed on the display 70, the operator can easily recognize the positions of the objects. However, the DRR 200 and the DRR 300 may not be necessarily generated.

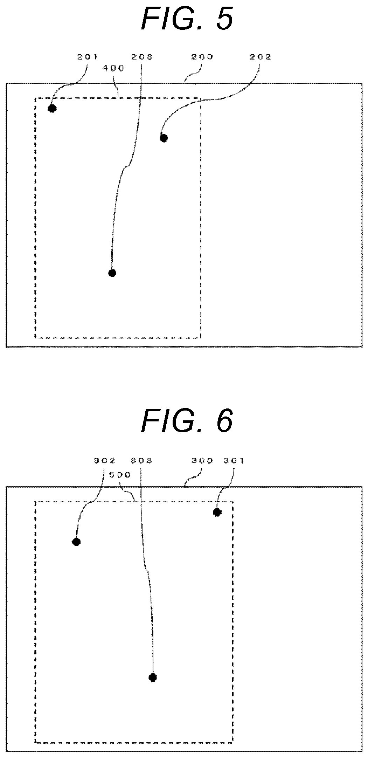

FIG. 5 is an example of the irradiation range on the DRR 200.

FIG. 6 is an example of the irradiation range on the DRR 300.

Regions where the positions of the objects in the fluoroscopic image are included are respectively calculated as the irradiation range of the X-ray 114a and 114b. For example, when the objects used as the tracking targets are three markers illustrated in FIG. 3 and FIG. 4, a region 400 in FIG. 5 covering all of the images 201, 202, and 203 of the markers in the DRR 200, and a region 500 in FIG. 6 covering all of the images 301, 302, and 303 of the markers in the DRR 300 are calculated as the irradiation range. When the graphics shown in FIG. 5 and FIG. 6 are displayed on the display 70, the operator can easily recognize the region 400 and the region 500 which are the irradiation range. Also when the object is an affected part, by displaying the DRR, the operator can easily recognize the irradiation range. However, since confirmation of the irradiation range by the operator is not necessary, generation and display of the DRR 200 and the DRR 300 are not necessary. When the fluoroscopic image is displayed, the operator can know the ranges to be irradiated with the X-ray 114a and 114b.

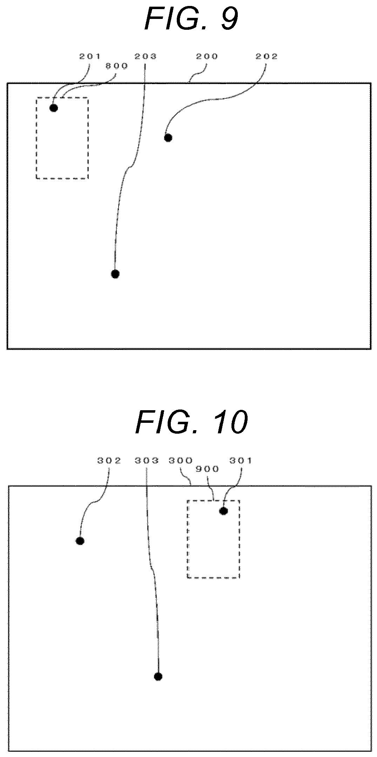

FIG. 9 is an example of the irradiation range in a case in which the object of the tracking target on the DRR 200 is one.

FIG. 10 is an example of the irradiation range in a case in which the object of the tracking target on the DRR 300 is one.

The number of objects used as the tracking target need not be plural. For example, when a marker corresponding to the images 201 and 301 in FIG. 3 and FIG. 4 is a tracking target, a region including the position thereof is calculated as the irradiation range. That is, regions 800 and 900 in FIG. 9 and FIG. 10 may be determined as the irradiation range.

As illustrated in FIG. 9 and FIG. 10, the operator can easily recognize the regions 800 and 900 as the irradiation range. Also when the obj ect is the affected part, by generating and displaying the DRR, the operator can easily recognize the irradiation range. However, since confirmation of the irradiation range by the operator is not necessary, generation and display of the DRR 200 and the DRR 300 are not necessary. When the fluoroscopic image is displayed, the operator can know the ranges to be irradiated with the X-ray 114a and 114b.

According to the information processing apparatus and a treatment system according to the present embodiment, a pre-capturing of the fluoroscopic image using X-ray is not necessary for determining the irradiation range. Therefore, a patient is less exposed to the radiation when determining the irradiation range for fluoroscopy for treatment.

Modification Example 1

The three-dimensional volume data may be moving image data. For example, the moving image may be 4D-CT image data. A plurality of CT images is included in 4D-CT images corresponding to the 4D-CT image data.

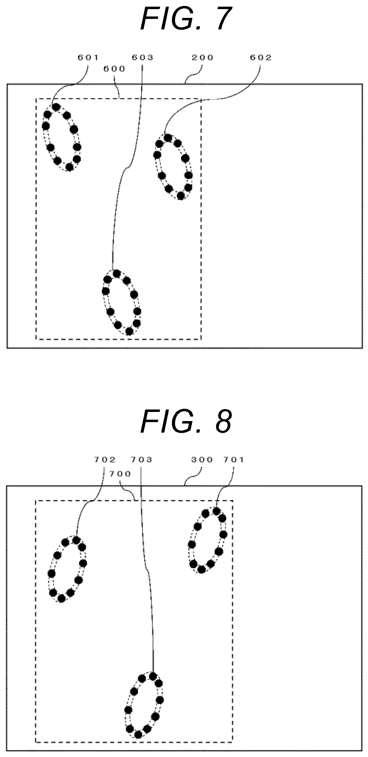

FIG. 7 schematically illustrates a moving image of the DRR 200 to explain a method of determining an irradiation range when the three-dimensional volume data is moving image data. FIG. 7 corresponds to a case in which the three-dimensional volume data includes ten CT images and three markers are located in the subject P. FIG. 7 illustrates trajectories 601, 602, and 603 of each marker in the moving image of the DRR 200 generated from the CT image. Black points in FIG. 7 respectively indicate the center of each marker which is detected.

FIG. 8 illustrates trajectories 701, 702, and 703 of each marker in the moving image of the DRR 300 in a same condition as that in FIG. 7.

As the irradiation range, a region 600 including the trajectories 601, 602, and 603 in the moving image of the DRR 200, and a region 700 including the trajectories 701, 702, and 703 in the moving image of the DRR 300 are determined. Here, it is assumed that each of the objects is the marker, but the object may be an affected part.

In addition, as illustrated in FIG. 7 and FIG. 8, when the trajectories 601, 602, 603, 701, 702, and 703 of the object, and the irradiation regions 600 and 700 are both included in each of the DRR 200 and the DRR 300 that are displayed on the display 70, the operator can easily recognize the trajectories and the irradiation regions. For example, the trajectories and the irradiation regions may be displayed between Steps S23 and S24 in FIG. 2. However, since the prior confirmation of the irradiation range by the operator is not necessary, the display of the irradiation range in the DRR 200 and the DRR 300 is not necessary. When the fluoroscopic image is displayed, the operator can know the range to be irradiated with the X-rays 114a and 114b.

Modification Example 2

The condition for fluoroscopic imaging measured by the determination section 100b may be at least one of irradiation intensity of the X-rays 114a and 114b, irradiation energy of the X-rays 114a and 114b, an output voltage of the X-ray sources 112a and 112b, and an output current of the X-ray sources 112a and 112b.

As described above, the position of the object in the three-dimensional volume space is specified. Paths of the X-ray 114a and 114b are calculated using the geometry information. In addition, the DRR 200 and the DRR 300 are calculated using the calculated paths of the X-rays 114a and 114b. Image quality of the DRR 200 and the DRR 300 depends on the output voltage and the output current of the X-ray sources 112a and 112b which are virtually set. In other words, when the output voltage and the output current of the X-ray sources 112a and 112b are virtually set to certain values, the DRR 200 and the DRR 300 having certain image quality can be generated. When the tube voltages of the X-ray sources 112a and 112b are changed, intensity and energy of the X-ray 114a and 114b are changed, and thus contrast and an amount of noise of the fluoroscopic image are changed. When the output current of the X-ray sources 112a and 112b are changed, the intensity of the X-ray 114a and 114b are changed, and thus the amount of noise of the fluoroscopic image is changed. The output current or the output voltage can be respectively set for each of the X-ray sources 112a and 112b.

The position of the object in the DRR 200 and the DRR 300 may be calculated using the geometry information. Then, the output voltage or the output current of the X-ray sources 112a and 11b at which a brightness contrast of the object with respect to peripheries of the object is large and the amount of the noise is small may be determined as a condition for fluoroscopic imaging. For example, the irradiation intensity or the output current of the X-ray sources 112a and 112b is increased, as an attenuation rate of the X-ray 114a and 114b passing through the object or peripheries of the object increases, so that the amount of noise around the object in the fluoroscopic image is reduced. However, when the irradiation intensity or the output current of the X-ray sources 112a and 112b is increased, an amount of radiation exposure on the patient is increased. For that reason, the irradiation intensity or the output current should have an upper limit.

When the three-dimensional volume data is the CT image data, the attenuation rate can be calculated based on an integrated value of CT values of the X-ray 114a and 114b in the three-dimensional volume data. Alternatively, the intensity or energy of the X-rays 114a and 114b may be calculated based on the output voltage or the output current of the X-ray sources 112a and 112b, and at least one thereof may be determined as the condition for fluoroscopic imaging.

The condition for fluoroscopic imaging may be a combination of any two or more of the image capturing range, the irradiation intensity of the X-rays 114a and 114b, the irradiation energy of the X-rays 114a and 114b, and the output voltage of the X-ray sources 112a and 112b, and the output current of the X-ray sources 112a and 112b in the first embodiment. According to the modification example, an appropriate condition for fluoroscopic imaging can be determined.

Modification Example 3

A respiratory sensor (not illustrated) may be set on the subject P, and sensor information therefrom may be used to determine the condition for fluoroscopic imaging. The respiratory sensor monitors a breathing phase of the subject P. The breathing phase includes an expiratory phase, an intake phase, and a phase therebetween. As a correspondence between the sensor information and the condition for fluoroscopic imaging is set in advance, the determination section 100b can determine the condition for fluoroscopic imaging from the correspondence and the sensor information.

When the condition for fluoroscopic imaging includes the irradiation range, at the time of capturing the 4D-CT image in advance, the respiratory sensor is set on the subject P, and the correspondence between the sensor information and the position of the object is calculated in advance. Then, the irradiation range is determined from the position of the object, the geometry information, and the sensor information. As a result, the irradiation range suitable for each breathing phase of the subject P can be determined.

When the condition for fluoroscopic imaging includes at least one of the irradiation intensity of the X-ray 114a and 114b, the irradiation energy of the X-ray 114a and 114b, the output voltage of the X-ray sources 112a and 112b, and the output current the X-ray sources 112a and 112b, at the time of capturing the 4D-CT image, the respiratory sensor is set on the subject P, and the correspondence between the sensor information and the position of the object is calculated. The output voltage and the output current of the X-ray sources 112a and 112b, at which the brightness contrast of the object with respect to peripheries of the object in the DRR 200 and the DRR 300 becomes a maximum at each breathing phase expressed by the sensor information are calculated. At least any one thereof is determined as the condition for fluoroscopic imaging. According to the present modification example, the condition for fluoroscopic imaging suitable for each breathing phase can be calculated.

Modification Example 4

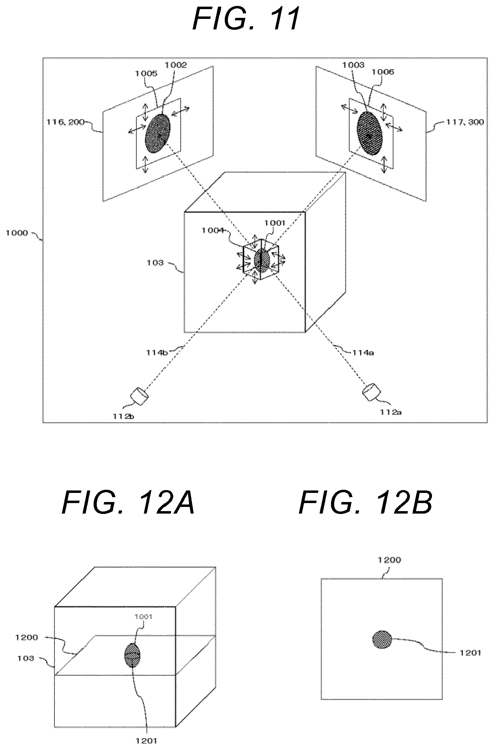

FIG. 11 exemplifies a screen 1000 displayed on the display 70.

The screen 1000 includes a space 103 corresponding to the three-dimensional volume data, the DRR 200, and the DRR 300. The operator may input a correction instruction of the irradiation range through the input device 60. The DRR 200 and the DRR 300 are virtually-generated fluoroscopic images by the X-ray detectors 116a and 116b, which are generated from the three-dimensional volume data. In this example, the DRR 200 and the DRR 300 are displayed on the X-ray detectors 116a and 116b. Regions 1005 and 1006 including images 1002 and 1003 of the object 1001 in the three-dimensional volume data, respectively, indicate the irradiation range. A three-dimensional region 1004 in the space 103 corresponds to the regions 1005 and 1006. The operator can instruct to change width or height of each region by operating arrows displayed on the screen 1000 using the input device 60 in the regions 1004, 1005, and 1006. The determination section 100b receives the instruction of the operator, modifies each region, and displays each modified region on the display 70. Since the regions 1004, 1005, and 1006 correspond to each other, when the operator instructs a modification of a size of any one of the regions, the determination section 100b may also change a size of the other corresponding regions at the same time. An interface for instructing the change by the operator may not limited to a mouse. For example, when a display portion is a touch panel, the operator can operate the touch panel with fingers. A numeral value such as a width, height, or depth of the regions 1004, 1005, and 1006 may be displayed on the screen 1000, and the operator may change the values using a keyboard.

According to the present example, the operator can easily recognize the irradiation range. In addition, the regions 1004, 1005, and 1006 automatically determined by the determination section 100b can be changed manually by the operator with simple operations.

When the three-dimensional volume data is moving image data, a frame image of each breathing phase may be displayed to the operator. By looking at an image of each breathing phase displayed on the display 70, the operator can instruct to adjust each of the regions 1004, 1005, and 1006, and the determination section 100b can therefore receive the instruction to adjust each region. That is, the modification example 3 can be combined with the present modification example.

(Second Embodiment)

The radiation therapy system according to a second embodiment has the same configuration as that in FIG. 1, but a processing function of the processing circuit 100 is partially different. Differences from the first embodiment will be described hereinafter.

The acquisition section 100a of the processing circuit 100 acquires the contour of the object in a three-dimensional space corresponding to the three-dimensional volume data of the subject P. Here, the acquisition section 100a of the processing circuit 100 may acquire the position of the object from the storage circuit 50 or outside the radiation therapy system 10. Alternatively, the acquisition section 100a of the processing circuit 100 may calculate the contour of the object from the three-dimensional volume data.

The determination section 100b of the processing circuit 100 determines the conditions 1108 and 1109 for fluoroscopic imaging of the fluoroscopic imaging device 30 from the contour and the fluoroscopic image each predetermined period of time, and transfers the conditions to the image capturing control section 100e.

The image capturing control section 100e of the processing circuit 100 the fluoroscopic imaging device 30 such that the fluoroscopic imaging device 30 captures the fluoroscopic image of the subject P each predetermined period of time. Each of the fluoroscopic images is captured according to the condition for fluoroscopic imaging.

That is, the procedure of the present embodiment is different from that of the first embodiment illustrated in FIG. 2 in that a step to be returned to when the determination at Step S29 in FIG. 2 is NO is Step S23. In addition, the procedure of the present embodiment is different in that at the time of obtaining the condition for fluoroscopic imaging in Step S23, the fluoroscopic image captured in Step S24 is used.

FIG. 12A illustrates an example of a contour of an object in space corresponding to the three-dimensional volume data. FIG. 12B illustrates an example of the contour on an axial cross-section 1200.

The contour is for example, a set of a plurality of contours 1201 of the object 1001 in each of the axial cross-sections 1200. The contour 1201 of the object 1001 in each of the axial cross-sections 1200 is, for example, input by the operator when the treatment plan is created.

The determination section 100b determines the condition for fluoroscopic imaging at each timing, for example, in the following manner. First, a center position of the object is detected from the fluoroscopic image using any of various kinds of image processing technology. Next, a three-dimensional contour of the object is calculated based on the center position thereof and the contour. Next, the contour of object in the fluoroscopic image is calculated based on the three-dimensional contour of the object and the geometry information. Finally, a region including the contour in the fluoroscopic image is set, and the region is determined as the irradiation range.

Since the irradiation range in each frame of the fluoroscopic image is calculated, a range narrower than the irradiation range including the trajectory exemplified in the first embodiment can be calculated. Therefore, according to the information processing apparatus and the treatment system according to the present embodiment, radiation exposure of a patient can be reduced.

Moreover, the computer or the embedded system in the above embodiments is used for executing each process in the embodiments described above based on a program stored in the recording medium, and may be a device including one such as a personal computer or a microcomputer, a system in which a plurality of devices are connected through a network, and the like.

In addition, the computer in the above embodiments is not limited to a personal computer, and includes an arithmetic processing apparatus including an information processing equipment, and a microcomputer, and is collectively referred to as an apparatus which are capable of performing functions in the above embodiments by a program.

According to the radiation therapy system or the information processing apparatus according to at least one embodiments described above, the radiation for treatment can be appropriately emitted while suppressing radiation exposure of a patient.

While certain arrangements have been described, these arrangements have been presented by way of example only, and are not intended to limit the scope of the claims. Indeed, the device and the system described herein may be embodied in a variety of other forms; furthermore, various omissions, substitutions and changes in the form of the device and the system described herein may be made.

Further embodiments:

E1. A processing device (20) for a radiation device (30), that is configured to carry out the steps of:

retrieving, from a data storage (50), volume data of a subject that was generated by imaging an internal structure of the subject;

determining a position of an object in the subject based on the retrieved volume data of the subject;

obtaining geometry information including information to project position of the object from three-dimensional space onto two-dimensional space of image by a radiation source (112a, 112b) and a radiation detector (116a, 116b); and

determining a condition for imaging with the radiation source, so that the object can be captured through the imaging, based on the volume data, the position of the object, the geometry information.

E2. The processing device according to item E1, wherein the steps further comprise:

controlling a stage (111) on which the subject is placed to be positioned at a predetermined position; and

controlling at least one of the radiation source and a collimator (118a, 118b) in accordance with the condition, the collimator being between the radiation source and the subject.

E3. The processing device according to item E1, wherein the steps further comprise:

determining a precise position of the object based on an image of the object; and

controlling at least one of the radiation source and the collimator in accordance with the precise position.

E4. The processing device according to item E1, wherein

the condition includes an irradiation range of the radiation for the imaging.

E5. The processing device according to item E1, wherein the steps further comprise:

based on the volume data and the geometry information, calculating an projected position of the object that is projected onto two-dimensional space of image by the radiation source; and

narrowing the irradiation range, so that the projected position is included in the narrowed irradiation range for the imaging.

E6. The processing device according to item E4, wherein the steps further comprise:

receiving a detection value of a respiratory sensor attached to the subject; and

retrieving, from the data storage that stores correspondence between a plurality of detection values and a plurality irradiation ranges, the irradiation range corresponding to the detection value.

E7. The processing device according to item E4, wherein

the volume data include a plurality of volume data frames that was generated in accordance with a periodical movement of the subject,

the position of the object in the subject is determined for each of the plurality of volume data frames, and

the irradiation range is determined, so that positions of the object corresponding to the plurality of volume data frames are included in the irradiation range for the imaging.

E8. The processing device according to item E1, wherein

the condition includes at least one of intensity of radiation, radiation energy, a value of current supplied to the radiation source, and a value of voltage applied to the radiation source.

E9. The processing device according to item E8, wherein the steps further comprise:

based on the volume data, the position of the object, and the geometry information, calculating an estimated attenuation rate of radiation that is virtually irradiated on the object, wherein at least one of the intensity of radiation, the radiation energy, the value of current supplied to the radiation source, and the value of voltage applied to the radiation source is increased as the estimated attenuation rate increases.

E10. The processing device according to item E1, wherein the steps further comprise:

receiving a user input made on an input device (60) ; and

modifying the determined condition based on the user input.

E11. The processing device according to item E1, wherein the steps further comprise:

controlling a display (70) to display a graphical user interface including at least one of a three-dimensional image of the subject corresponding to the volume data and a two-dimensional digitally reconstructed radiograph (DRR) of the subject generated based on the volume data, and the geometry information.

E12. The processing device according to item E1, wherein the steps further comprise:

controlling an imaging device (30) for the imaging; and
determining a center position of the object based on an image obtained by the imaging, wherein a three-dimensional contour of the object is determined as the position of the object based on the volume data, and
the condition is determined based on the center position of the object and the three-dimensional contour of the object.

E13. A radiation therapy system (10), comprising:

a stage (111) on which a subject is to be placed;
an imaging device (30) including a radiation source (112a, 112b) that emits radiation for imaging and a radiation detector (116a, 116b);
a treatment radiation source (120);
a data storage (50); and
a processing device (100) that carries out steps of:

retrieving, from the data storage (50), volume data of the subject that was generated by imaging an internal structure of the subject;
determining a position of an object in the subject based on the retrieved volume data of the subject;
obtaining geometry information including information to project position of the object from three-dimensional space onto two-dimensional space of image by the radiation source and the radiation detector; and
determining a condition of imaging with the radiation source, so that the object can be captured through the imaging, based on the volume data, the position of the object, and the geometry information.

E14. The radiation therapy system according to item E13, wherein the steps further comprise:

controlling the stage to be positioned at a predetermined position; and
controlling at least one of the radiation source and a collimator (118a, 118b) in accordance with the condition, the collimator being disposed between the radiation source and the subject.

E15. The radiation therapy system according to item E14, wherein the steps further comprise:

determining a precise position of the object based on an image of the object; and

controlling at least one of the radiation source and the collimator in accordance with the precise position.

E16. The radiation therapy system according to item E13, wherein
the condition includes an irradiation range of the radiation for the imaging.

E17. The radiation therapy system according to item E16, wherein the steps further comprise:

based on the volume data, the position of the object, and the geometry information, calculating an projected position of the object that projected onto two-dimensional space of image by the radiation source; and
narrowing the irradiation range, so that the projected position is included in the narrowed irradiation range of the imaging.

E18. The radiation therapy system according to item E16, wherein
the volume data include a plurality of volume data frames that was generated in accordance with a periodical movement of the subject,
the position of the object in the subject is determined for each of the plurality of volume data frames, and
the irradiation range is determined, so that positions of the object corresponding to the plurality of volume data frames are included in the irradiation range for the imaging.

E19. The radiation therapy system according to item E13, wherein
the condition includes at least one of intensity of radiation, radiation energy, a value of current supplied to the radiation source, and a value of voltage applied to the radiation source.

E20. The radiation therapy system according to item E19, wherein the steps further comprise:

based on the volume data, the position of the object, and the geometry information, calculating an estimated attenuation rate of the radiation that is virtually irradiated on the object, wherein at least one of the intensity of radiation, the radiation energy, the value of current supplied to the radiation source, and the value of voltage applied to the radiation source is increased as the estimated attenuation rate increases.

E21. The radiation therapy system according to item E13, wherein the steps further comprise:

controlling the stage on which the subject is placed to be positioned at a predetermined position; and
controlling an imaging control circuit (113) that is configured to control positions of the radiation source, a collimator, and the radiation detector, the collimator being between the radiation source and the subject.

E22. The processing device according to item E1, wherein
the geometry information includes at least one of a position of the radiation source, a position of the radiation detector, and a direction of the radiation detector.

**Claims**

1. A processing device (20) for a radiation device (30), that is configured to carry out the steps of:

   retrieving, from a data storage (50), volume data of a subject that was generated by imaging an internal structure of the subject;
   determining a position of an object in the subject based on the retrieved volume data of the subject;
   obtaining geometry information including information to project position of the object from three-dimensional space onto two-dimensional space of image by a radiation source (112a, 112b) and a radiation detector (116a, 116b); and
   determining a condition for imaging with the radiation source, so that the object can be captured through the imaging, based on the volume data, the position of the object, the geometry information.

2. The processing device according to claim 1, wherein the steps further comprise:

   controlling a stage (111) on which the subject is placed to be positioned at a predetermined position; and
   controlling at least one of the radiation source and a collimator (118a, 118b) in accordance with the condition, the collimator being between the radiation source and the subject.

3. The processing device according to claim 1, wherein the steps further comprise:

   determining a precise position of the object based on an image of the object; and
   controlling at least one of the radiation source and the collimator in accordance with the precise position.

4. The processing device according to claim 1, wherein the condition includes an irradiation range of the radiation of the radiation for the imaging.

5. The processing device according to claim 1, wherein the steps further comprise:

   based on the volume data and the geometry information, calculating an projected position of the object that is projected onto two-dimensional space of image by the radiation source; and
   narrowing the irradiation range, so that the projected position is included in the narrowed irradiation range for the imaging.

6. The processing device according to claim 4, wherein the steps further comprise:

   receiving a detection value of a respiratory sensor attached to the subject; and
   retrieving, from the data storage that stores correspondence between a plurality of detection values and a plurality irradiation ranges, the irradiation range corresponding to the detection value.

7. The processing device according to claim 4, wherein the volume data include a plurality of volume data frames that was generated in accordance with a periodical movement of the subject, the position of the object in the subject is determined for each of the plurality of volume data frames, and the irradiation range is determined, so that positions of the object corresponding to the plurality of volume data frames are included in the irradiation range for the imaging.

8. The processing device according to claim 1, wherein the condition includes at least one of intensity of radiation, radiation energy, a value of current supplied to the radiation source, and a value of voltage applied to the radiation source.

9. The processing device according to claim 8, wherein the steps further comprise:

   based on the volume data, the position of the object, and the geometry information, calculating an estimated attenuation rate of radiation that is virtually irradiated on the object, wherein at least one of the intensity of radiation, the radiation energy, the value of current supplied to the radiation source, and the value of voltage applied to the radiation source is increased as the estimated attenuation rate increases.

10. The processing device according to claim 1, wherein the steps further comprise:

receiving a user input made on an input device (60) ; and

modifying the determined condition based on the user input.

11. The processing device according to claim 1, wherein the steps further comprise:

controlling a display (70) to display a graphical user interface including at least one of a three-dimensional image of the subject corresponding to the volume data and a two-dimensional digitally reconstructed radiograph (DRR) of the subject generated based on the volume data, and the geometry information.

12. The processing device according to claim 1, wherein the steps further comprise:

controlling an imaging device (30) for the imaging; and

determining a center position of the object based on an image obtained by the imaging, wherein

a three-dimensional contour of the object is determined as the position of the object based on the volume data, and

the condition is determined based on the center position of the object and the three-dimensional contour of the object.

13. A radiation therapy system (10), comprising:

a stage (111) on which a subject is to be placed;

an imaging device (30) including a radiation source (112a, 112b) that emits radiation for imaging and a radiation detector (116a, 116b);

a treatment radiation source (120);

a data storage (50); and

a processing device (100) that carries out steps of:

retrieving, from the data storage (50), volume data of the subject that was generated by imaging an internal structure of the subject;

determining a position of an object in the subject based on the retrieved volume data of the subject;

obtaining geometry information including information to project position of the object from three-dimensional space onto two-dimensional space of image by the radiation source and the radiation detector; and

determining a condition of imaging with the radiation source, so that the object can be captured through the imaging, based on the volume data, the position of the object, and the geometry information.

14. The radiation therapy system according to claim 13, wherein the steps further comprise:

controlling the stage to be positioned at a predetermined position; and

controlling at least one of the radiation source and a collimator (118a, 118b) in accordance with the condition, the collimator being disposed between the radiation source and the subject.

15. The radiation therapy system according to claim 14, wherein the steps further comprise:

determining a precise position of the object based on an image of the object; and

controlling at least one of the radiation source and the collimator in accordance with the precise position.

## FIG. 1

# FIG. 2

```
                    ┌─────────┐
                    │  START  │
                    └────┬────┘
                         ▼
          ┌──────────────────────────┐
          │        ACQUIRE           │  S20
          │  THREE-DIMENSIONAL       │
          │     VOLUME DATA USED     │
          │   FOR TREATMENT PLAN     │
          └────────────┬─────────────┘
                       ▼
          ┌──────────────────────────┐
          │  ACQUIRE POSITION OF     │  S21
          │       OBJECT IN          │
          │   THREE-DIMENSIONAL      │
          │         SPACE            │
          └────────────┬─────────────┘
                       ▼
          ┌──────────────────────────┐
          │    ACQUIRE GEOMETRY      │  S22
          │     INFORMATION OF       │
          │   FLUOROSCOPIC IMAGE     │
          │    CAPTURING DEVICE      │
          └────────────┬─────────────┘
                       ▼
          ┌──────────────────────────┐
          │  DETERMINE CONDITION     │  S23
          │    OF FLUOROSCOPIC       │
          │     IMAGING DEVICE       │
          └────────────┬─────────────┘
                       ▼
          ┌──────────────────────────┐
          │        CAPTURE           │  S24
          │   FLUOROSCOPIC IMAGE     │
          └────────────┬─────────────┘
                       ▼
          ┌──────────────────────────┐
          │   DETECT POSITION OF     │  S25
          │         OBJECT           │
          └────────────┬─────────────┘
                       ▼
   YES          ◇──────────────────◇  S26
          ◇  IS POSITION OF          ◇
   ◇ OBJECT WITHIN PREDETERMINED ◇
          ◇     REGION?             ◇
                       │ NO
 ┌──────────────────┐  │
 │ RADIATE RADIATION│  │   S28
 │  FOR TREATMENT   │  │
 └──────────────────┘  ▼
              ◇──────────────────◇  S29
          ◇  IS THERE FINISHING    ◇ ── NO
          ◇    INSTRUCTION?        ◇
                       │ YES
                       ▼
                  ┌─────────┐
                  │   END   │
                  └─────────┘
```

## FIG. 3

## FIG. 4

## FIG. 5

201 203 200 202
400

## FIG. 6

302 303 300 301
500

## FIG. 7

601   603   200   602
600

## FIG. 8

702   703   300   701
700

## FIG. 9

## FIG. 10

## FIG. 11

## FIG. 12A

## FIG. 12B

Europäisches
Patentamt

European
Patent Office

Office européen
des brevets

## EUROPEAN SEARCH REPORT

Application Number

EP 17 15 7599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/071798 A1 (KONINKL PHILIPS NV [NL]) 21 May 2015 (2015-05-21) * abstract; figures 2,10 * * page 4, line 9 - page 8, line 25 * ----- | 1-15 | INV. A61N5/10 A61B6/00 |
| A | WO 2008/057166 A2 (ACCURAY INC [US]; MAURER CALVIN R [US]; FU DONGSHAN [US]; KAHN ROBERT) 15 May 2008 (2008-05-15) * figures 1A,2 * ----- | 1-15 | |
| A | WO 2012/007036 A1 (BRAINLAB AG [DE]; FEILKAS THOMAS [DE]) 19 January 2012 (2012-01-19) * abstract * ----- | 1-15 | |

TECHNICAL FIELDS
SEARCHED      (IPC)

A61N
A61B

The present search report has been drawn up for all claims

| Place of search | Date of completion of the search | Examiner |
|---|---|---|
| Munich | 4 July 2017 | Kajzar, Anna |

EPO FORM 1503 03.82 (P04C01)

## ANNEX TO THE EUROPEAN SEARCH REPORT
## ON EUROPEAN PATENT APPLICATION NO.

EP 17 15 7599

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2017

| Patent document cited in search report | | Publication date | Patent family member(s) | | Publication date |
|---|---|---|---|---|---|
| WO 2015071798 | A1 | 21-05-2015 | CN 105744891 A | | 06-07-2016 |
| | | | EP 3071108 A1 | | 28-09-2016 |
| | | | US 2016287201 A1 | | 06-10-2016 |
| | | | WO 2015071798 A1 | | 21-05-2015 |
| WO 2008057166 | A2 | 15-05-2008 | CN 101553281 A | | 07-10-2009 |
| | | | EP 2061559 A2 | | 27-05-2009 |
| | | | JP 2010508895 A | | 25-03-2010 |
| | | | US 2008130825 A1 | | 05-06-2008 |
| | | | US 2011116703 A1 | | 19-05-2011 |
| | | | WO 2008057166 A2 | | 15-05-2008 |
| WO 2012007036 | A1 | 19-01-2012 | EP 2593922 A1 | | 22-05-2013 |
| | | | US 2013094742 A1 | | 18-04-2013 |
| | | | WO 2012007036 A1 | | 19-01-2012 |

EPO FORM P0459

For more details about this annex : see Official Journal of the European Patent Office, No. 12/82